# EUROPEAN PATENT SPECIFICATION

(11) **EP 1 883 314 B1**
(45) Date of publication and mention of the grant of the patent: **09.09.2009**
(21) Application number: 06747652.3
(22) Date of filing: 23.05.2006
(51) Int. Cl.: A23K 1/16, A23K 1/14, A23K 1/18

(54) **A FISH FEED CONTAINING SOYA PROTEIN**
FISCHFUTTER MIT SOJAPROTEIN
ALIMENT POUR POISSONS CONTENANT DES PROTEINES DE SOJA

(30) Priority: 26.05.2005 NO 20052532; 16.05.2006 NO 20062199
(43) Date of publication of application: 06.02.2008
(73) Proprietor: TROUW INTERNATIONAL B.V., NL-5831 JN Boxmeer (NL)
(72) Inventor: KOPPE, Wolfgang, M., N-4011 Stavanger (NO)
(74) Representative: Hatt, Anna Louise
(86) International application number: PCT/NO2006/000193
(87) International publication number: WO 2006/126889

(56) References cited:
- EP-A- 1 474 997
- US-A1- 2003 219 468
- US-B1- 6 217 915
- YUEMING DERSJANTI-LI: "The use of soy protein in aquafeeds" AVANCES EN NUTRICIÓN ACUICOLA VI. MEMORIAS DEL VI SIMPOSIUM INTERNACIONAL DE NUTRICIÓN ACUICOLA, 3 September 2002 (2002-09-03), - 6 September 2002 (2002-09-06) pages 541-557, XP003000351 Cancún, Quintana Roo, México
- LOURENS DE WET: "Use of soya in aquafeeds" PAPER PRESENTED DURING AFAM'S ANNUAL SYMPOSIUM, 13 August 2004 (2004-08-13), pages 10-13, XP003000350
- A KROGDAHL, A.M BAKKE-MCKELLEP, K.H. ROED, G. BAEVERFJORD: "Feeding Atlantic salmon Salmo salar L. soybean products: effects on disease resistance (furunculosis), and lysozyme and IgM levels in the intestinal mucose" AQUACULTURE NUTRITION, vol. 6, 2000, pages 77-84, XP002480968
- BJERKENG B. ET AL.: 'Cholesterol and short-chain fatty acids in diets for Atlantic salmon Salmo salar (L.): effects on growth, organ indices, macronutrient digestibility, and fatty acid composition' AQUACULTURE NUTRITION vol. 5, 1999, pages 181 - 191, XP003000349
- DE WET L.: 'Use of soya in aquafeeds' THIS PAPER WAS PRESENTED DURING AFMA'S ANNUAL SYMPOSIUM 13 August 2004, pages 10 - 13, XP003000350
- YUEMING DERSANG-LI: 'The Use of Soy Protein in Aquafeeds' ADVANCES EN NUTRICION ACUICOLA VI. MEMORIAS DEL VI SIMPOSIUM INTERNACIONAL DE NUTRICION ACUICOLA 03 September 2002 - 06 September 2002, CANCUN, QUINTANA ROO, MEXICO, pages 542 - 558, XP003000351

## Description

The invention relates to an animal feed, in particular a feed for fish, the feed containing an additive component. The invention also relates to an animal feed, in particular a feed for fish, the feed containing known amounts of soya protein and an additive component preventing or reducing the occurrence of soya-induced enteritis (intestinal inflammation). The invention also relates to an animal feed, in particular a feed for fish, the feed having an elevated soya products content relative to what has earlier been defensible to use, and an additive component preventing enteritis or reducing enteritis to an acceptable level.

The additive component is butyric acid or a known salt of butyric acid or a known ester of butyric acid or a combination of butyric acid, salts of butyric acid and esters of butyric acid.

The invention also relates to the use of such a feed for feeding fish, particularly salmonoids.

Aquaculture has had a considerable global success and is, today, an important protein source for the world's increasing population. There is also an increasing knowledge that the consumption of fish *per capita* should be increased because of the positive health effects. At the same time, it is no longer possible to increase the amount of fish caught in the wild, because the stocks cannot take a larger reduction. For some stocks of wild fish the catch will have to be reduced in order for the fish to be sustainable, and some stocks of wild fish have collapsed already. Therefore, aquaculture will be of constantly increasing importance as a supplier of fish to the world's population.

Fish, especially carnivorous fish, need protein, fat, minerals and vitamins in order to grow and to be in good health. Originally, in the farming of carnivorous fish, there were used whole fish or ground fish to cover the nutrient requirements of the farmed fish. Ground fish mixed with dry raw materials of various kinds, such as fish meal and starch, was termed soft feed. Gradually, the farming became industrialized and soft feed was replaced by dry feed of the pressed feed type. The pressed feed was gradually replaced by dry feed of the extruded feed type. Today extruded feed is nearly universal in the farming of a number of fish species such as several kinds of salmonoid, cod, sea bass and sea bream.

After the yolk sack has been used up, marine fry require small feed particles in the size of 80 µm and larger. Fry of salmonoids can eat larger particles from approximately 500 µm and larger. Such small feed particles can be produced by agglomeration or it may be a granulate produced by crushing and sifting larger feed particles. These larger feed particles may be produced by pressing or extrusion.

The dominant protein source in dry feed for fish has been fish meal of different qualities. The fish meal may be of North-European origin or of South-American origin, but on a world-wide basis also other types of fish meal are used. Other animal protein sources are also used. Thus, it is known to use blood meal, bone meal, feather meal and other types of meal produced from other slaughterhouse waste, for example chicken meal. It is also known to use vegetable protein such as wheat gluten, maize gluten, soya protein, lupine meal, pea meal, rape meal, sunflower meal and rice flour.

In the same way as the catch of wild fish for consumption may no longer be increased, the catch of so-called industrial fish may not be increased either. Industrial fish is a raw material for the production of fish meal and fish oil. The global production of fish meal and fish oil is dominated by the fishery taking place off the coasts of Peru and Chile. The total quantity of these countries fluctuate somewhat from one year to the next. At about 7 years' intervals the weather phenomenon El Niño occurs. During an "E1 Niño" the caught amount drops relatively much. This affects the availability of fish meal and fish oil on the world market, and the prices rise considerably for these raw materials.

The aquaculture industry and especially the fish feed industry have predicted for several years that there will be a shortage of both fish meal and fish oil in the future. In addition there has been a wish to find a cheaper alternative to fish meal and fish oil. Slaughterhouse waste, such as blood meal, bone meal, feather meal and chicken meal are good protein sources. However, in some geographic regions, such as Europe, there has been a prohibition against using such raw materials in the production of feeds for food-producing animals and fish.

Vegetable protein raw materials, such as soya, rape, wheat gluten, maize gluten, lupines, peas, sunflower and rice, have been put to use as raw materials for fish feed. Soya is a raw material with a high protein content and a favourable price and is available in very large quantities on a world-wide basis. Therefore, soya has been used in feeds for many years, also in fish feeds.

It is well known that soya contains a great number of substances which are called, by a generic term, antinutrients. These antinutrients comprise, among other substances, trypsin inhibitor, phytin, glycinin, β-conglycinin, lecithin, oligo saccharids and saponin. Some of these antinutrients cause intestinal reactions like inflammations (enteritis), but it has still not been fully clarified what substances trigger these reactions. Generally, it is true that antinutrients reduce growth, health and well-being. This applies to several food-producing animals, especially young animals like calves and piglets, but it also applies to fish, and then to carnivorous fish in particular. For fish, particularly salmonoids, the soya content must not exceed 5-15 % (of wet weight) in the diets in order for soya-induced enteritis to be prevented (Krogdahl et al. 2003: Effects of graded levels of standard soybean meal on intestinal structure, mucosal enzyme activities, and pancreatic response in Atlantic salmon (Salmo salar L.). Aquaculture Nutrition, 9, 361-371).

It is well known that soya-induced enteritis is found in the distal portions of the intestine of salmon fish (Krogdahl et. al. 2003). The distal portion of the fish intestine is also called the rear intestine of the fish.

V. Denstadli et al., Aquaculture 240, 2004, p. 385-398 describe the absorption of fatty acids in the intestine of salmon, proving that medium long and long-chain fatty acids are absorbed in the forward portions of the intestine.

In spite of a long-felt need for using more soya in feeds for some food-producing animals, including fish, especially salmonoids, and in spite of the great research activity that there has been in this field due to the considerable possible economic gains, it has not been possible until now to produce a commercial feed for salmonoids, having a soya content exceeding 5-15 %, and at the same time being competitive in terms of price.

Short-chain fatty acids and their salts have been added to feeds for several purposes. Thus, it is known that formic acid and salts of formic acid can stimulate growth. It is also known for short-chain fatty acids to be used as a substitute for antibacterial growth promoters. This includes the use of formic acid, acetic acid, propionic acid, lactic acid, sorbinic acid, fumaric acid, malic acid, tartaric acid and citric acid.

Butyric acid is a short-chain fatty acid. Butyric acid is produced by the bacteria in the intestine as carbohydrates are broken down. Butyric acid is produced together with lactic acid, acetic acid and propionic acid. In most warm-blooded animals butyric acid is the short-chain fatty acid present in the lowest concentration. For example, in the large intestine of pigs acetic acid, propionic acid and butyric acid are present in the ratio of 53:34:10 (Bach Knudsen et al. 1991: Gastrointestinal implications in pigs of wheat and oat fractions. Br. J. Nutr. 65, 233-248). Correspondingly, it is known for short-chain fatty acids to be present in the fermentation in the intestine of herbivorous fish, but also in the intestines of carnivorous fish like "red sea bream" (Chrysophrys major). In salmon (Salmo salar) it has been established that such bacterial fermentation is much lower and that there is little of short-chain fatty acids in the intestine (Holben et al. 2002: Phylogenetic analysis of intestinal microflorea indicates a novel Mycoplasma phylotype in farmed and wild salmon. Microb. Ecol. 44, 175-185).

It is known that butyric acid is the preferred energy source for the coloncytes of the intestine. Thus, butyric acid has an important function in the intestine in intestinal growth, regeneration and nutrient absorption. Butyric acid prevents inflammatory reactions in the intestine by modifying transcription factors. The transcription factors control the expression of inflammatory response genes (Andoh and Fujiyama. 2004: Anti-inflammatory roles of dietary fiber and short-chain fatty acids as regards inflammatory bowel diseases. AgroFOOD Industry Hi-Tech, January-February 2004, 42). Butyric acid also plays an important role against intestinal cancer. It has been shown that butyric acid induces differentiation and apoptosis in cancerous cells (Hague et al. 1996: The role of butyrate in human colonic epithelial cells: an energy source or inducer of differentiation and apoptosis? Proceedings of the Nutrition Society, 55, 937-943).

For many years there has been intense research on the possibility of increasing the soya content in feeds for food-producing animals, also in feeds for fish. The research has been carried out by universities, suppliers of soya and by the feed industry itself.

There are several qualities of soya that can be used in feeds. Whole soya beans may be used, but they are not used for fish feeds due to the high antinutrients content. Full-fat soya is heat-treated soya beans, and such have been used to a certain extent. The heat treatment inactivates some of the known antinutrients. The most used soya product in the fish feed industry is so-called extracted soya. Extracted soya is described as a protein product containing 44 % or 48 % protein. It is produced by crushing, heating and flaking soya beans. The fat content in the conditioned flakes is reduced by the use of hexan or corresponding hydrocarbon solvents as means of extraction. The extracted flakes are boiled and marketed as such, or they are marketed as ground meal. Extracted soya is available in two qualities, so-called Lopro soya, which has a relatively low protein content, and so-called Hipro soya, which has a relatively high protein content.

So-called ethanol-extracted soya protein contains little antinutrients. Ethanol-extracted soya is sold as "soya protein concentrate". Soya protein concentrate does not induce enteritis and is thus advantageous for use in feeds, including fish feeds, but this product is expensive and the price prevents the use for this purpose. There is also commercially available a product called Hamlet soya which is low in antinutrients. It has not been published how Hamlet soya protein is produced. Larger amounts of Hamlet soya protein in the feed will also induce enteritis in salmonoids.

Most refined is the so-called soya protein isolate. This is used for baby food, among other things.

Table 1 gives an survey of the nutrients and antinutrients content in various qualities of soya products.

**Table 1: Various soya protein products**

| | **Whole soya beans** | **Flour of soya bean** | **Soya protein concentrate enzyme-treated** | **Soya protein concentrate alcohol-extracted** | **Soya protein isolate** | **Hamlet protein** |
|---|---|---|---|---|---|---|
| Water (%) | 10-12 | 10-12 | 6.5 | 7 | 6.5 | 8.0 |
| Raw protein (%) | 35.5 | 42-50 | 57.5 | 65 | 90 | 55.0 |
| Fat (%) | 19 | 1-1.5 | 2.5 | 1 | 1 | 2.5 |
| Ash (%) | 4.7 | 5.5-6 | 6.8 | 6 | 5 | 6.8 |
| Urease-activity (pH-rise) | 2.0 | 0.05-0.5 | <0.05 | <0.05 | <0.05 | |
| Trypsin inhibitor (mg/g) | 45-50 | 1-8 | 1 | 2 | <1 | 1 |
| Glycinin (ppm) | 180,000 | 66,000 | <100 | <100 | | |
| β-conglycinin (ppm) | >60,000 | 16,000 | <10 | <10 | | 2 |
| Lecitins (ppm) | 3,500 | 10-200 | <1 | <1 | 0 | <1 |
| Oligo saccharids (%) | 14 | 15 | 1 | 3 | 0 | |
| Saponins (%) | 0.5 | 0.6 | 0 | 0 | 0 | |

| | | | | | | |
|---|---|---|---|---|---|---|
| Source: Peisker. 2001. Manufacturing of soy protein concentrate for animal nutrition. Conference Title: Feed manufacturing in the Mediterranean region. Improving safety: from feed to food. Proceedings of the III Conference of Feed Manufacturers of the Mediterranean, organized by ASFAC, 22-24 March, 2000, in Cahiers Options Mediterraneennes vol. 54, 103-107 ISSN: 1022-1379 Editors: Brufau, J. Publisher: Centre International des Hautes Etudes Agronomiques Mediterraneennes Paris, France). Data sheet for Hamlet soya protein. | | | | | | |

Butyric acid is used today in nutritional and clinical connection. In most cases the bacteria of the intestine are stimulated to produce more butyric acid. In clinical cases direct administration of butyric acid can be used on damaged intestinal segments (Young and Gibson. 1995: i Physiological and clinical aspects of short-chain fatty acids. Editors: Cummings, J.H., Rombeau, J.L., Sakata T., Cambridge University Press 1995, ISBN 0-521-61613-1).

Salts of short-chain fatty acids are known to be used in fish feeds in experimental connections. Thus, Bjerkeng et al. have published a piece of work, in which the effects of cholesterol and salts of short-chain fatty acids on growth and digestibility were examined. (Bjerkeng et al. 1999: Cholesterol and short-chain fatty acids in diets for Atlantic salmon Salmo salar (L.): effects on growth, organ indices, macronutrient digestibility, and fatty acid composition. Aquaculture Nutrition 5, 181-191). The experiment was carried out in 1995/96 and the feed that was used, was a complete fish feed consisting of fish meal, fish oil, wheat, vitamins, minerals and astaxanthin. It did not contain soya protein or other vegetable proteins with the exception of protein from wheat. The purpose of the experiment was to study the effect of cholesterol in fish feeds and whether a mixture of sodium salts from acetic acid, propionic acid and butyric acid would affect possible effects of the cholesterol. To the feed there was added either 0, 0.04 or 0.17 % salt of butyric acid.

Ethanol-extracted soya protein, Hamlet soya protein and soya protein isolate are good protein sources, but the processing makes these protein sources considerably more expensive than soya beans and soya flour. The price may exceed the price of fish meal. In addition, available quantities of these soya proteins are too small to satisfy the requirements of the feed industry.

The first object of the invention is to reduce or prevent the development of soya-induced enteritis by the use of a fish feed containing between 5 % and 15 % soya protein on a wet weight basis. A second object is to increase the amount of soya protein in fish feeds to more than 15 % on a wet weight basis, without inducing enteritis. A third object is to increase the amount of soya in fish feeds to more than 15 % on a wet weight basis, without the degree of enteritis exceeding the degree of enteritis observed when soya protein is used in amounts of between 5 % and 15 % in fish feeds without the use of an additive component.

It is a further object to provide a fish feed with soya protein without it inducing production sufferings like soya-induced enteritis, while, at the same time, the growth is just as good as with a fish feed in which the major part of the protein comes from fish meal or from other protein sources that do not induce enteritis.

The invention also relates to the use of such fish feeds.

Surprisingly, it has turned out that the admixture of small amounts of an additive component in fish feeds overcomes the drawbacks of the soya protein in the form of soya-induced enteritis in the rear intestine when soya protein is used in fish feeds, in particular in feeds for salmonoids.

The additive component is butyric acid, a known salt of butyric acid or a mixture of butyric acid and salt of butyric acid.

Thus it is particularly astonishing that the additive component, which is a short-chain fatty acid, is not absorbed until it has arrived in the rear intestine, taking into consideration that medium long and long fatty acids are absorbed in the forward portion of the intestine. Thus is anticipated that a short-chained fatty acid like butyric acid is absorbed in the portions of the intestine, too.

In what follows, there are described non-limiting examples of experimental results when using the invention, the results being visualized in the accompanying figures, in which:
- Figure 1: shows the distribution of individual enteritis values for each diet in Example 1;
- Figure 2: shows the distribution of individual enteritis values for each diet in Example 2; and
- Figure 3: shows the distribution of enteritis values sorted into diets in Example 3.

The degree of soya-induced enteritis in fish is determined by histology. A piece of the front part of the rear intestine of the fish is cut out and fixed in buffered formol in a known manner. Tissue sections are made from the fixed material and are dyed with haematoxylin and eosin. The degree of enteritis is determined by a scale ranging from 1 to 5. The value 1 is given to a completely normal intestine without any damage. The value 2 is given for very weakly developed enteritis. The value 3 is given for slightly developed enteritis. The value 4 is given for developed and serious enteritis and the value 5 is given for highly developed enteritis. The degree of enteritis can also be stated in "half" values, such as 1.5 and 2.5. In practice the values 1 and 2 mean that the fish is completely healthy. Separate studies have shown that fish having enteritis values lower than 3, grow in a normal way. Enteritis values of 3 and above involves that the fish will grow more slowly. Enteritis values of 5 are hardly seen as this value is considered to be lethal.

### Description of action/use

### Example 1

For the experiment two basic diets were made: A control diet composed of 62.5 kg fish meal, 18.3 kg wheat, 19.0 kg fish oil and 0.4 kg vitamins/minerals/pigment. The second basic diet was composed of 52.9 kg fish meal, 15.0 kg extracted soya, 12.4 kg wheat, 19.6 kg fish oil and 0.4 kg vitamins/minerals/pigment.

The control diet consisting of fish meal was called FM in Example 1. The diet containing soya was called SO. Based on the SO diet, three diets were made, containing different amounts of short-chain fatty acids:
- Diet FA consisting of diet SO + 0.5 % formic acid
- Diet LA consisting of diet SO + 0.75 % lactic acid
- Diet BA consisting of diet SO + 0.005 % butyric acid

The percentages are based on the wet weight of the SO feed.

The diets were of the extruded type.

The experiment was carried out on salmon (Salmo salar) in sea water. The water temperature was constantly 7.6 °C. The average weight of the fish was 3 kg. One experimental vessel of 3 metres in diameter was allocated to each diet. The number of fish in each vessel varied from 54 to 58. The salmon were distributed into the vessels and then fed the FM diet for 24 days before the experimental feeds were fed for 34 days. There was no mortality in the experiment. At the end of the experiment 10 fish per vessel were killed and samples of the rear intestine were removed for histological examination.

A chemical analysis of the diets is given in Table 2:

**Table 2. Chemical analysis of the five diets of Example 1**

| **Diet** | **Dry solids (%)** | **Protein (%)** | **Fat (%)** |
|---|---|---|---|
| FM | 94.1 | 46.4 | 25.6 |
| SO | 95.1 | 46.4 | 25.5 |
| FA | 94.9 | 46.2 | 26.2 |
| LA | 93.9 | 45.9 | 25.9 |
| BA | 93.3 | 45.8 | 25.8 |

Intestinal samples were taken from 10 fish per diet. Average enteritis values are given in Table 3. The results have been stated with standard deviations.

**Table 3. Average enteritis values for the different diets of Example 1.**

| **Diet** | **Average enteritis value** |
|---|---|
| FM | 1.0 ± 0.0 ^{a} |
| SO | 1.9 ± 0.8 ^{bc} |
| FA | 2.1 ± 0.8 ^{c} |
| LA | 1.8 ± 0.7 ^{bc} |
| BA | 1.4 ± 0.2 ^{ab} |

The pure fish meal diet (FM) had an enteritis value of 1.0, which was expected. Diet SO had a value of 1.9, and this was significantly higher than for diet FM. Diets FA and LA gave enteritis values at the same level as diet SO, whereas diet BA gave a reduction in enteritis value. The enteritis value of diet BA is not significantly different from those of diet FM and diet SO, but it still shows a positive tendency (Duncan test, p<0.05).

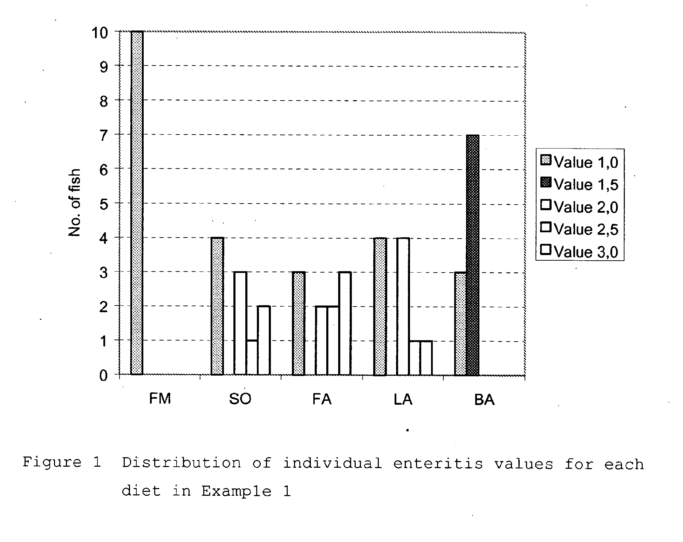

In order to have a more detailed look at.the development in enteritis values, the distribution of the individual values is shown in Figure 1.

In Figure 1 it can be seen more clearly that the distribution of enteritis values changes when butyric acid is added to the soya diet. The diets SO, FA and LA all have a fairly similar distribution of enteritis values from the value 1.0 to 3.0, whereas for diet BA a clear shift can be seen in the values, to values 1 and 1.5. The values of 2.0, 2.5 and 3.0 which are found for diet SO, have disappeared in diet BA. This shows that the addition of 0.005 % butyric acid in the diet with 15 % soya has a clear positive effect on intestinal inflammation caused by soya.

### Example 2

The experiment was done to verify the positive effect of butyric acid from Example 1. In Example 1 relatively little enteritis was found in the fish that were given 15 % soya. In this experiment, to ensure that enteritis would be induced, soya molasses was used instead of soya. Soya molasses is the fraction which is removed from soya by ethanol extraction in the production of soya protein concentrate. It is well known that soya molasses induces enteritis (van den Ingh et al. 1996: Alcohol-soluble components in soybeans cause morphological changes in the distal intestine of Atlantic salmon, Salmo salar L. Journal of Fish Diseases, 19, 47-53, and Krogdahl et al. 2000: Feeding Atlantic salmon Salmo salar L. soybean products: effects on disease resistance (furunculosis), and lysozyme and IgM levels in the intestinal mucosa. Aquaculture Nutrition, 6, 77-84).

Two diets were made, based on the same formula: 62.6 kg fish meal, 12.3 kg wheat, 19.0 kg fish oil, 0.4 kg vitamins/minerals/pigment and 10 kg soya molasses. Diet MO was the basic feed and diet BA2 was the basic feed with 0.01 % butyric acid added. In the form of antinutrients a molasses content of approximately 10 % corresponds to a soya content of 30-40 % (extracted soya). The diets were of the extruded type.

The experiment was conducted on small salmon (0.4 kg) in sea water at 8.2 °C. One experimental vessel of 1 metre in diameter was allocated to each diet. There were 40 salmon in each vessel. The fish were distributed into the vessels and then fed the experimental feed for 27 days. There was not mortality in the experiment.

At the end of the experiment samples of the rear intestine were removed for examination. Five samples from each diet were examined histologically, as previously described.

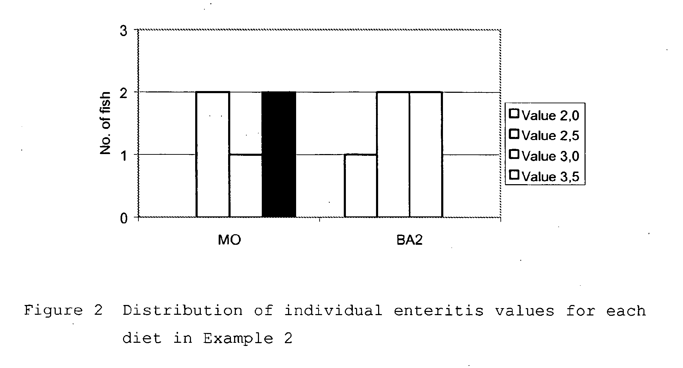

Average enteritis values were 3.0 for diet MO and 2.6 for diet BA2. The distribution of the enteritis values is shown in Figure 2.

Also in this experiment the diet with butyric acid added shows that the distribution of enteritis values shifts towards lower values. For diet BA2, the value 3.5 is gone and one fish has been given the value 2.0. This is very positive as a value above 3 indicates developed intestinal inflammation.

### Example 3

The experiment was done to find the optimum ratio of admixture of butyric acid in a feed for salmon, the feed having a high extracted soya content.

Six diets were made, based on the same formula with approximately 25 % soya, but with increasing admixture of butyric acid (0 - 0.1 - 0.07 - 0.13 - 0.19 - 0.25 %). The basic recipe contained 45.8 kg fish meal, 13.1 kg wheat, 19.0 kg fish oil, 0.4 kg vitamins/minerals/pigment and 25 kg extracted soya. A diet based on fish meal was also included as a positive reference (diet FM'). This diet contained 55.8 kg fish meal, 32.7 kg wheat, 15.0 kg fish oil, 0.4 kg vitamins/minerals/pigment. The diet codes are given in Table 4. The diets were of the extruded type.

**Table 4. Diet codes for Example 3**

| **Diet** | **Formula** |
|---|---|
| FM' | Fish meal |
| SO' | Soya |
| BU01 | SO' + 0.01 % butyric acid |
| BU07 | SO' + 0.07 % butyric acid |
| BU13 | SO' + 0.13 % butyric acid |
| BU19 | SO' + 0.19 % butyric acid |
| BU25 | SO' + 0.25 % butyric acid |

The experiment was conducted on small salmon (0.34 kg) in sea water at 8.2 °C. Diets FM' and SO' were tested in four vessels each, whereas the remaining diets were tested in duplicate vessels. There were 25 salmon in each vessel. The fish were distributed into the vessels and then fed the experimental feed for 69 days.

At the end of the experiment, 8 or 16 fish per diet were killed and samples of the rear intestine were removed for histological examination (Table 5).

**Table 5. Survey of the removal of fish for histological examination**

| **Diet** | **Number of vessels per diet** | **Number of fish for histology per vessel** |
|---|---|---|
| FM' | 4 | 4 |
| SO' | 4 | 4 |
| BU01 | 2 | 8 |
| BU07 | 2 | 8 |
| BU13 | 2 | 4 |
| BU19 | 2 | 4 |
| BU25 | 2 | 4 |

A chemical analysis of the experimental diets is shown in Table 6.

**Table 6. Chemical analysis of the experimental diets of Example 3.**

| **Diet** | **Protein (%)** | **Fat (%)** | **Water (%)** |
|---|---|---|---|
| FM' | 42.9 | 21.4 | 5.8 |
| SO' | 46.5 | 25.7 | 5.2 |
| BU01 | 46.0 | 24.6 | 5.3 |
| BU07 | 46.2 | 25.1 | 5.1 |
| BU13 | 46.0 | 24.6 | 5.1 |
| BU19 | 46.1 | 23.9 | 5.7 |
| BU25 | 46.2 | 23.7 | 5.3 |

The chemical analysis showed that the diets having approximately 25 % soya were all alike in the amounts of protein, fat and water. Diet FM', on the other hand, differed in protein and fat content to make an isoenergetic and isonitrogenic diet.

Average specific daily growth is shown in Table 6 together with standard deviations. Diet BU25 gave significantly lower growth than diet FM', diet SO' and the diets with up to 0.13 % butyric acid added (Duncantest, p<0.05).

**Table 7. Average specific daily growth in Example 3**

| **Diet** | **Specific daily growth (%/day)** |
|---|---|
| FM' | 0.91 ± 0.04 ^{b} |
| SO' | 0.88 ± 0.05 ^{b} |
| BU01 | 0.91 ± 0.03 ^{b} |
| BU07 | 0.85 ± 0.06 ^{b} |
| BU13 | 0.83 ± 0.09 ^{b} |
| BU19 | 0.80 ± 0.11 ^{ab} |
| BU25 | 0.68 ± 0.06 ^{a} |

Table 8 shows average enteritis values.

**Table 8. Average enteritis values**

| **Diet** | **Average enteritis value** |
|---|---|
| FM' | 1.0 |
| SO' | 3.0 |
| BU01 | 2.3 |
| BU07 | 2.7 |
| BU13 | 3.3 |
| BU19 | 2.9 |
| BU25 | 2.6 |

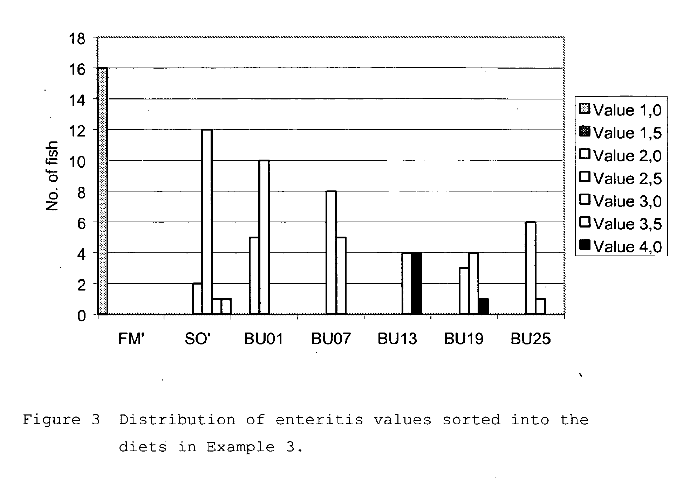

There is a clear reduction in the enteritis value from diet SO' to diet BU01. There is also a reduction for diet BU07, but for higher levels of butyric acid (BU13 and BU19) this reduction is not seen. To examine the development of enteritis value, the distribution of the individual values sorted into diets is shown in Figure 3. Here it is shown clearly that values of 3.0 and higher have disappeared when there was an addition of 0.01 % butyric acid (diet BU01). With an addition of 0.07 % butyric acid, there are 5 fish having a value of 3.0 but no fish having a higher value for this diet.

The three experiments show a clear positive effect on intestinal inflammation in salmon when a low dose of butyric acid is added to feeds with soya. In contrast, a high dose of butyric acid, 0.25 %, shows a negative effect on growth with about 25 % soya in the feed (Table 7).

### Alternative embodiments

The invention is not limited to butyric acid, but also comprises known salts of butyric acid. Besides being added as solutions, such salts may also be mixed with the dry raw materials. The salts may also be available in separate mixtures suitable for mixing with the rest of the dry raw materials.

The invention also includes known esters of butyric acid and mixtures of such known butyric acid esters. Methyl butyrate, ethyl butyrate and pentyl butyrate are non-limiting examples of suitable butyric acid esters according to the scope of the invention. Besides being added in their liquid form, such esters may also be added in the dry form, the butyric acid ester being a coating on dry particles, such as silica particles. In their dry form the butyric acid esters are mixed with the dry raw materials.

It is not previously known to use butyric acid as an additive component in fish feeds. Further, it is not known to add butyric acid or salts of butyric acid or butyric acid esters to fish feeds containing soya protein. In particular it is not known to add butyric acid or salts of butyric acid or butyric acid esters to fish feeds containing between 5 and 15 per cent by weight of soya protein, calculated on a wet weight basis. Further, it is not known to add butyric acid or salts of butyric acid or butyric acid esters to fish feeds containing more than 15 per cent by weight of soya protein, calculated on a wet weight basis. It is a surprising effect that the addition of small amounts of butyric acid and/or salts of butyric acid and/or butyric acid esters to fish feeds containing soya protein, prevents or reduces the occurrence of soya-induced enteritis (intestinal inflammation) and that the degree of enteritis becomes less serious.

It is also new that the amounts of soya products in fish feeds can be increased beyond a level equivalent to 15 % soya protein products with respect to antinutrients, without the occurrence of enteritis increasing or the seriousness of the enteritis increasing.

### TEXT IN FIGURES:

| **Figure** | **Reference** | **English text** |
|---|---|---|
| | Antall fisk | Number of fish |
| | Verdi | Value |
| | | Figure 1. Distribution of individual enteritis values for each diet in Example 1. |
| | | Figure 2. Distribution of individual enteritis values for each diet in Example 2. |
| | | Figure 3. Distribution of enteritis values sorted into the diets in Example 3. |

## Claims

1. A fish feed, **characterized in that** the fish feed contains soya protein and that in the fish feed there is added butyric acid or salts thereof or esters thereof.

2. The fish feed in accordance with claim 1, **characterized in that** the fish feed is of an agglomerated, granulated, pressed or extruded type.

3. The fish feed in accordance with claim 2, **characterized in that** the soya protein content in the fish feed is up to 15 per cent by weight, calculated on the basis of the wet weight of the total amount of feed ingredients included.

4. The fish feed in accordance with claim 2, **characterized in that** the soya protein content in the fish feed exceeds 15 per cent by weight, calculated on the basis of the wet weight of the total amount of feed ingredients included.

5. The fish feed in accordance with claim 1, **characterized in that** the butyric acid content is between 0.005 and 0.25 per cent by weight or equivalent amounts of salts of butyric acid or equivalent amounts of esters of butyric acid, calculated on the basis of the wet weight of the total amount of feed ingredients included.

6. The fish feed in accordance with claim 1, **characterized in that** the butyric acid is added to the fish feed in a non-dissociated form.

7. The fish feed in accordance with claim 1, **characterized in that** the salt of butyric acid is added to the fish feed in a solid form or in a saline solution or in a combination of these.

8. The fish feed in accordance with claim 1, **characterized in that** the butyric acid ester is added to the fish feed in the liquid form, or in a coating of butyric acid ester on dry particles or in a combination of these.

9. The fish feed in accordance with claim 1, **characterized in that** the butyric acid ester is methyl butyrate, ethyl butyrate or pentyl butyrate, or a mixture of at least two of the said esters.

10. Butyric acid/or salts thereof or esters thereof for use in a fish feed containing soya protein to prevent or reduce the occurrence of, or reduce the seriousness of, soya-induced enteritis.

11. Butyric acid or salts thereof or esters thereof for use in a fish feed in accordance with claim 10, **characterized in that** the butyric acid content is between 0.005 and 0.25 per cent by weight or equivalent amounts of salts of butyric acid or equivalent amounts of esters of butyric acid, calculated on the basis of the wet weight of the total amount of feed ingredients included.

## Patentansprüche

1. Fischfutter, **dadurch gekennzeichnet, daß** es Sojaprotein enthält und dem Fischfutter Buttersäure oder Salze oder Ester davon zugesetzt sind.

2. Fischfutter nach Anspruch 1, **dadurch gekennzeichnet, daß** es in zusammengeballter, granulierter, verpresster oder extrudierter Form vorliegt.

3. Fischfutter nach Anspruch 2, **dadurch gekennzeichnet, daß** der Sojaproteingehalt im Fischfutter bis zu 15 Gew.-%, bezogen auf das Naßgewicht der Gesamtmenge an enthaltenen Futterkomponenten, beträgt.

4. Fischfutter nach Anspruch 2, **dadurch gekennzeichnet, daß** der Sojaproteingehalt im Fischfutter 15 Gew.-%, bezogen auf das Naßgewicht der Gesamtmenge an enthaltenen Futterkomponenten, übersteigt.

5. Fischfutter nach Anspruch 1, **dadurch gekennzeichnet, daß** der Buttersäuregehalt 0,005 bis 0,25 Gew.-% oder äquivalente Mengen der Salze der Buttersäure oder äquivalente Mengen der Ester der Buttersäure, bezogen auf das Naßgewicht der Gesamtmenge an enthaltenen Futterkomponenten, beträgt.

6. Fischfutter nach Anspruch 1, **dadurch gekennzeichnet, daß** die Buttersäure dem Fischfutter in nichtdissoziierter Form zugesetzt ist.

7. Fischfutter nach Anspruch 1, **dadurch gekennzeichnet, daß** das Salz der Buttersäure dem Fischfutter in fester Form oder als Salzlösung oder als Kombination aus beiden zugesetzt ist.

8. Fischfutter nach Anspruch 1, **dadurch gekennzeichnet, daß** der Buttersäureester dem Fischfutter in flüssiger Form oder als Beschichtung aus Buttersäureester auf Trockenpartikeln oder als Kombination aus beiden zugesetzt ist.

9. Fischfutter nach Anspruch 1, **dadurch gekennzeichnet, daß** der Buttersäureester Methyl-, Ethyl- oder Pentylbutyrat oder ein Gemisch aus wenigstens zwei dieser Ester ist.

10. Buttersäure oder Salze oder Ester davon zur Verwendung in einem Sojaprotein enthaltendem Fischfutter zur Verhinderung oder Verminderung des Auftretens von sojainduzierter Enteritis oder zur Verminderung des Schweregrades von sojainduzierter Enteritis.

11. Buttersäure oder Salze oder Ester davon zur Verwendung in einem Fischfutter nach Anspruch 10, **dadurch gekennzeichnet, daß** der Buttersäuregehalt 0,005 bis 0,25 Gew.-% oder äquivalente Mengen der Salze der Buttersäure oder äquivalente Mengen der Ester der Buttersäure, bezogen auf das Naßgewicht der Gesamtmenge an enthaltenen Futterkomponenten, beträgt.

## Revendications

1. Aliment pour poissons, **caractérisé en ce que** l'aliment pour poissons contient des protéines de soja et que l'aliment pour poissons contient en outre de l'acide butyrique ou des sels ou esters de celui-ci.

2. Aliment pour poissons selon la revendication 1, **caractérisé en ce que** l'aliment pour poissons est de type aggloméré, granulé, comprimé ou extrudé.

3. Aliment pour poissons selon la revendication 2, **caractérisé en ce que** la teneur en protéine de soja dans l'aliment pour poissons va jusqu'à 15 % en poids, calculés sur la base du poids sec de la quantité totale d'ingrédients de l'aliment inclus.

4. Aliment pour poissons selon la revendication 2, **caractérisé en ce que** la teneur en protéines de soja dans l'aliment pour poissons dépasse 15 % en poids, calculés sur la base du poids sec de la quantité totale d'ingrédients de l'aliment inclus.

5. Aliment pour poissons selon la revendication 1, **caractérisé en ce que** la teneur en acide butyrique se situe entre 0,005 et 0,25 % en poids ou en quantités équivalentes de sels d'acide butyrique ou en quantités équivalentes de d'esters d'acide butyrique, calculés sur la base du poids sec de la quantité totale d'ingrédients de l'aliment inclus.

6. Aliment pour poissons selon la revendication 1, **caractérisé en ce que** l'acide butyrique est ajouté à l'aliment pour poisson sous une forme non dissociée.

7. Aliment pour poissons selon la revendication 1, **caractérisé en ce que** le sel d'acide butyrique est ajouté à l'aliment pour poissons sous une forme solide ou dans une solution physiologique ou en combinaison de celles-ci.

8. Aliment pour poissons selon la revendication 1, **caractérisé en ce que** l'ester d'acide butyrique est ajouté à l'aliment pour poissons sous forme liquide ou dans un revêtement d'ester d'acide butyrique sur des particules sèches ou en combinaison de ceux-ci.

9. Aliment pour poissons selon la revendication 1, **caractérisé en ce que** l'ester d'acide butyrique est le méthylbutyrate, l'éthylbutyrate ou le pentylbutyrate ou un mélange d'au moins deux desdits esters.

10. Acide butyrique ou sels de celui-ci ou esters de celui-ci à utiliser dans un aliment pour poissons, contenant des protéines de soja pour prévenir ou réduire l'occurrence d'entérite induite par le soja ou en réduire la gravité.

11. Acide butyrique ou sels de celui-ci ou esters de celui-ci, à utiliser dans un aliment pour poissons selon la revendication 10, **caractérisé en ce que** la teneur en acide butyrique se situe entre 0,005 et 0,25 % en poids ou en quantités équivalentes de sels d'acide butyrique ou en quantités équivalentes d'esters d'acide butyrique, calculés sur la base du poids sec de la quantité totale d'ingrédients de l'aliment inclus.
